Europäisches Patentamt

(19) European Patent Office

Office européen des brevets

(11) Publication number: **0 272 365**
**B1**

(12) **EUROPEAN PATENT SPECIFICATION**

(45) Date of publication of patent specification: **29.08.90**

(51) Int. Cl.⁵: **C 07 C 323/58**

(21) Application number: **87106729.4**

(22) Date of filing: **08.05.87**

(54) Method of producing L-cystine.

(30) Priority: **19.11.86 JP 273913/86**

(43) Date of publication of application:
**29.06.88 Bulletin 88/26**

(45) Publication of the grant of the patent:
**29.08.90 Bulletin 90/35**

(84) Designated Contracting States:
**CH DE FR GB IT LI NL**

(56) References cited:
**DE-A-3 613 388**
**GB-A-1 486 146**

**CHEMICAL ABSTRACTS, vol. 83, no. 5, 04 Aug
1975, Columbus, OH (US); J.T.SNOW et al.:
"Oxidation of sulfhydryl groups to disulfides by
sulfoxydes", p. 547, no. 43720r**

(73) Proprietor: **MITSUI TOATSU CHEMICALS INC.**
**No. 2-5, Kasumigaseki 3-chome**
**Chiyoda-Ku Tokyo 100 (JP)**

(72) Inventor: **Miyahara, Shyoichiro**
**Hirabaru-machi 300**
**Omuta-shi Fukuoka-ken (JP)**
Inventor: **Kamiguchi, Toshiaki**
**Yamashita-machi 35**
**Omuta-shi Fukuoka-ken (JP)**
Inventor: **Miyahara, Tooru**
**Kunugi 1471-2**
**Omuta-shi Fukuoka-ken (JP)**
Inventor: **Hashimukai, Tadashi**
**Kogane-machi 2-13**
**Omuta-shi Fukuoka-ken (JP)**
Inventor: **Nitta, Kazunari**
**Oaza-misaki 1874-28**
**Omuta-shi Fukuoka-ken (JP)**

(74) Representative: **Thomsen, Dieter, Dr.rer.nat.**
**Kaiser-Ludwig-Platz 6**
**D-8000 München 2 (DE)**

Courier Press, Leamington Spa, England.

## Description

### Background of the invention

### 1. Field of the invention

This invention relates to a method of producing L-cystine by an enzyme reaction.

### 2. Description of the prior art

Cystine can be easily reduced to cysteine, L-cystine and L-cysteine are amino acids containing sulfur and are used as, for example, medicines or their raw materials, or additives to food or cosmetics. It is particularly worthy of notice that they have recently come to be used in large quantities for making cold hair waving solutions.

There are known a variety of methods for producing L-cysteine. They include (1) extraction from natural substances, (2) organic synthesis, (3) fermentation, and (4) enzyme reaction. The extraction of L-cysteine from natural substances has the disadvantages that no stable supply of raw materials can be expected, and that the extracted product is likely to contain other unnecessary amino acids. The organic synthesis of L-cysteine requires its separation from D-cysteine. The method of producing L-cysteine by fermentation is undesirably low in efficiency. Therefore, none of these three methods is appropriate for industrial application.

There are known a number of methods for producing L-cysteine by employing enzymes. They include (1) synthesis of L-cysteine from L-serine and hydrogen sulfide by employing cysteine synthase or desulfhydrase, (2) synthesis from e.g. substituted β-alanine and a metal sulfide by employing cysteine desulfhydrase, and (3) synthesis from 2-amino-thiazoline-4-carboxylic acid (ATC) by employing L-ATC-hydrase, ATC-racemase or S-carbamyl-L-cysteine hydrase. Japanese Patent Application No. 84545/1985, which was filed by the assignee of this invention, discloses a method which produces L-cysteine by reacting L-serine and a compound having a sulfhydryl group, such as metal hydrosulfide, in the presence of tryptoplan synthase.

Whichever method may be employed, however, it is very difficult to separate cysteine directly from a reaction product, as the reaction product is complicated in composition, and as cysteine is highly soluble in water. Therefore, it is usual to oxidize cysteine to cystine and separate it from the reaction product. Refined cysteine is recovered by electrolytic reduction, etc.

There are known a number of methods for oxidizing cysteine to cystine. For example, Japanese Patent Publication No. 7634/1982 discloses a method which employs air or a peroxide, such as $H_2O_2$, for oxidizing cysteine in an aqueous product of fermentation, while maintaining it in a pH range of 5 to 10.

This method has, however, a number of drawbacks. It cannot be used for producing cystine from e.g. L-serine until after cysteine has been produced by the enzyme reaction. When cysteine is oxidized with air or $H_2O_2$, products of decom-

position other than cystine are also produced to a considerable extent unless the oxidizing conditions are strictly controlled.

For the enzyme reaction of L-serine, it is apparently better to use hydrogen sulfide ($H_2S$) directly as a commund containing a sulfhydryl group because of its high reactivity and low cost. However, as $H_2S$ gas is hardly soluble in water, it has been usual not to use it directly, but to use a water-soluble salt, e.g. sodium hydrosulfide, as a source of $H_2S$ supply.

### Summary of the invention

Under these circumstances, it is an object of this invention to provide an improved method of producing L-cystine from L-serine by an enzyme reaction.

According to a salient feature of this invention, dimethyl sulfoxide (DMSO) is used for oxidizing L-cysteine in an aqueous reaction product obtained by the enzyme reaction of L-serine with hydrogen sulfide, or an alkali metal hydrosulfide or sulfide. Dimethyl sulfoxide is employed in the system in which the enzyme reaction takes place, so that the cysteine which is produced may be successively converted to cystine. The use of DMSO also has the advantage of enabling the satisfactory use of hydrogen sulfide as it assists the dissolution of hydrogen sulfide in the aqueous medium for the enzyme reaction.

Moreover, DMSO does not hinder the enzyme reaction at all, but oxidizes L-cysteine to L-cystine successively, thereby contributing to accelerating the enzyme reaction drastically. This invention can, therefore, prevent the decomposition of L-cysteine forming any by-product and produce L-cystine from L-serine at a high yield.

### Detailed description of the invention

According to this invention, it is possible to use any enzyme that can be used for the enzyme reaction of L-serine with hydrogen sulfide, or an alkali metal hydrosulfide or sulfide. It is possible to use any known enzyme, such as cysteine synthase or desulfhydrase. It is also possible to use tryptophan synthase as proposed in the prior patent application which has hereinbefore been mentioned.

The use of tryptophan synthase is particularly effective and desirable. Various kinds of microorganisms can be used for producing the enzyme, as disclosed in Japanese Patent Application No. 84545/1985. They include *Escherichia coli* MT-10232 (FERM BP-19). *Escherichia coli* MT-10242 (FERM BP-20) and *Neurospora crassa* (ATCC 14692). It is particularly advantageous to use the tryptophan synthase obtained from *Escherichia coli*.

The enzyme need not necessarily be a pure product of extraction. It is possible to use a cultured product of any such microorganisms, living microorganisms collected therefrom by centrifugal separation, the frozen or freezed-dry product thereof, the microorganisms obtained by ultrasonic treatment, etc.

As regards the concentration of the substrate, L-serine, in the reaction solution, there is no limitation in particular, but it is usually advisable to use a solution having a concentration of 1 to 25% by weight. The amount of the enzyme which is added to the solution depends on varoius factors, such as the concentration of the substrate and the activity of the enzyme. It cannot be generally specified, but should be selected on a case to case basis. If tryptophan synthase is used, it is desirable to add a vary small amount of pyridoxal 5'-phosphate, too, as a coenzyme. The solution may, for example, contain 0.1 to 50 ppm of the acid.

The compound containing a sulfhydryl group which is employed for the enzyme reaction may be selected from among alkali metal hydrosulfides, such as NaHS and KHS, or alkali metal sulfides, such as $Na_2S$ and $K_2S$. Alternatively, it is possible to introduce hydrogen sulfide gas directly into the reaction system. In this case, it is advisable to employ at least one mol of hydrogen sulfide per mol of L-serine.

As regards the amount of DMSO, it is appropriate to use, say, 0.2 to 5.0 mols of DMSO per mol of L-serine. A range of 0.5 to 2.0 mols is particularly preferred. If only less than about 0.2 mol of DMSO is used for each mol of L-serine, it can hardly convert all of the L-cysteine product to L-cystine and if hydrogen sulfide is used, DMSO fails to assist its satisfactory dissolution. If more than about 5 mols of DMSO are employed, it is likely to inhibit the enzyme reaction seriously.

The reaction is usually carried out at a temperature of 20°C to 60°C in a pH range of 5 to 10 for a period of two to 50 hours. This period is preferably from two to ten hours if hydrogen sulfide is employed. As the reaction proceeds, the solution gradually presents a white color indicating the precipitation of L-cystine crystals.

The solution which has completed the reaction contains L-cystine and unreacted L-serine. The cystine can be separated from the reaction product by a method which will hereunder be described by way of example. The solution is heated in the presence of strong hydrochloric acid and active carbon and subjected to filtration under heat, whereby the cell residue is removed from the solution. Then, its pH is restored to about 5, whereby L-cystine is crystallized. An ordinary method of solid and liquid separation is, then, employed for collecting L-cystine of high purity. The L-cystine which has been collected can be reduced electrolytically to yield L-cysteine.

The invention will now be described more specifically with reference to several examples thereof. In each of the examples, the amount of cystine was calculated by determining the amount of cysteine in accordance with the known method of Gaitonde. The reaction product obtained in each example was diluted into a solution having a strength of 1/1000 to 1/2000. About the same quantity of 5 µM 1,4-dithiothreitol (reducing agent) was added to the solution. A 2N solution of NaOH was added to the solution until it had a pH of 8.0 to 8.5. The solution was left to stand at room temperature for an hour, whereby all of the cysteine which it contained was reduced into cysteine. An acidic ninhydrin reagent was added to the solution to develop the color of cysteine and its absorbance was measured at 560 nm by an absorptiometer. It was compared with a working curve which had previously been prepared to show the absorbance at 560 nm of a standard sample having a known concentration, whereby the concentration of cysteine in the product of each example was determined.

Example 1

A 200 ml separable flask, which was provided with a stirrer, a blow tube and an exhaust tube, was charged with 10 g of L-serine, 5.6 g of DMSO and 25 mg of pyridoxal 5'-phosphate. Deionized water was added to form 100 g of a solution in the flask. A 10% solution of NaOH was added to adjust the solution to a pH of 8.5. The flask was charged with 2.0 g (dry weight) of *Escherichia coli* MT-10242 (FERM BP-20) containing tryptophan synthase, while the solution was kept at a temperature of 35°C.

Hydrogen sulfide gas was blown from a bottle into the flask at a rate of about 14 ml per minute. The reaction was carried out by said blowing for about 4 hours while the pH value of the reaction solution was being maintained at pH 8.5 with a NaOH solution. The molar quantity of the hydrogen sulfide which was blown into the solution was about 1.5 times as large as that of L-serine. As the gas was blown, the solution gradually presented a white color indicating the precipitation of L-cystine.

Samples were taken as uniformly as possible from the reaction product. Each sample was dissolved in a 2N solution of hydrochloric acid. The bacteria were removed therefrom by centrifugal separation. The analysis of L-cystine in t4erms of L-cysteine gave a value of 9.3%. This meant that about 81% of L-serine has been converted to L-cystine.

About 25 ml of a 35% solution of hydrochloric acid were added to the reaction product to adjust it to a pH of 0.5. After 1.0 g of active carbon had been added, the solution was heated at 95°C for about an hour. Then, the solution was immediately subjected to filtration under heat, whereby the bacteria were removed therefrom. A solution having a transparent yellow color was obtained.

About 5 ml of a 40% solution of NaOH wre added to the solution to adjust it to a pH of 5. The solution was cooled to cause the precipitation of L-cystine crystals. The crystals were collected by a Buchner filter. They were washed in 20 ml of deionized water and were dried to yield 8.2 g of white L-cystine crystals. This amount corresponded to 72 mol% of the L-serine which had been used. The crystals showed a specific rotation of −218.7°, a purity of 99.1% and an ash content of 0.2%.

Example 2

A 200 ml separable flask provided with a stirrer was charged with 10 g of L-serine, 5.6 g of DMSO, 25 mg of pyridoxal 5'-phosphate and 9.5 g of sodium hydrosulfide (reagent of 70% purity). Deionized water was added to form 200 g of a solution. A 10% solution of NaOH was added to adjust the solution to a pH of 8.0. The flask was, then, charged with 2.0 g (dry weight) of the same bacteria as those which had been employed in Example 1. A phosphoric acid solution having a concentration of four mols per liter was added to keep the solution at a pH of 8.0, while it was kept at a temperature of 35°C. The reaction was continued for about 16 hours. The solution began to present a white color indicating the precipitation of cystine about two hours after the reaction had been started.

Samples were taken as uniformly as possible from the reaction product. Each sample was dissolved in a 2N solution of hydrochloric acid. The bacteria were removed therefrom by centrifugal separation. The analysis of L-cystine in terms of L-cysteine gave a value of 9.1%. This meant that about 79% of L-serine had been converted to L-cystine.

The procedure of Example 1 was thereafter repeated to yield 7.6 g of L-cystine crystals. This amount corresponded to 66.7 mol% of the L-serine which had been used. The crystals showed a specific rotation of −220°, a purity of 99.3% and an ash content of 0.1%.

Example 3

A 200 ml separable flask provided with a stirrer was charged with 10 g of L-serine, 5.6 g of DMSO, 25 mg of pyridoxal 5'-phosphate and 9.3 g of sodium sulfide (reagent of 96% or higher purity). Deionized water was added to form 200 g of a solution. A 10% HCl solution was added to adjust the solution to a pH of 8.0. The flask was charged with 2.0 g (dry weight) of the same bacteria as those which had been used in Example 1, while the solution was kept at a temperature of 35°C and a 10% HCl solution was added to maintain a pH of 8.0. The reaction was continued for about 16 hours. The solution began to present a white color indicating the precipitation of cystine about two hours after the reaction had been started.

Samples were taken as uniformly as possible from the reaction product. Each sample was dissolved in a 2N solution of hydrochloric acid. The bacteria were removed therefrom by centrifugal separation. The analysis of L-cystine in terms of L-crysteine gave a value of 8.7%. This meant that about 74% of L-serine had been converted to L-cystine.

The procedure of Example 1 was thereafter repeated to yield 7.4 g of L-cystine crystals. This amount corresponded to 64.9 mol% of the L-serine which had been used. The crystals showed a specific rotation of −221°, a purity of 98.9% and an ash content of 0.3%.

Example 4

A 200 ml separable flask provided with a stirrer was charged with 10 g of L-serine and 5.6 g of DMSO. Deionized water was added to form 50 g of a solution. The flask was, then, charged with an enzyme solution containing cysteine desulfhydrase which had been prepared by culturing *Bacillus subtilis* ATCC 6051 and subjecting the collected bacteria to ultrasonic disruption in a buffer. A solution of NaOH was added to form 100 g of a solution having a pH of 8.5.

Hydrogen sulfide gas was blown from a bottle into the flask at a rate of about 14 ml per minute. The reaction was carried out by said blowing for about 4 hours while the pH value of the reaction solution was being maintained at pH 8.5 with a NaOH solution. The molar quantity of the hydrogen sulfide which had been blown into the solution was about 1.5 times as large as that of L-serine. As the gas was blown, the solution gradually presented a white color indicating the presentation of L-cystine.

Samples were taken as uniformly as possible from the reaction product. Each sample was dissolved in a 2N solution of hydrochloric acid. The bacteria were removed therefrom by centrifugal separation. The analysis of L-cystine in terms of L-cysteine gave a value of 5.8%. This meant that about 53% of L-serine had been converted to L-cystine. The reaction product showed a cysteine concentration not exceeding 0.1%.

**Claims**

1. In a method of producing L-cystine from L-serine by reacting L-serine in the presence of an enzyme to form L-cysteine and oxidizing said L-cysteine, the improvement wherein said L-serine is reacted with a substance selected from the group consisting of hydrogen sulfide, alkali metal sulfides and alkali metal hydrosulfides and the reaction of said L-serine with said substance is carried out in the presence of dimethyl sulfoxide employed as an oxidizing agent for said L-cysteine.

2. A method as set forth in claim 1, wherein said enzyme is tryptophan synthase.

**Patentansprüche**

1. Verfahren zur Herstellung von L-Cystin aus L-Serin durch Umsetzung von L-Serin in Gegenwart eines Enzyms unter Bildung von L-Cystein und Oxidation des L-Cysteins, wobei das L-Serin mit einer Substanz aus der Gruppe von Schwefelwasserstoff, Alkalimetallsulfiden und Alkalimetallhydrogensulfiden umgesetzt wird und die Reaktion des L-Serins mit der betreffenden Substanz in Gegenwart von Dimethylsulfoxid, verwendet als Oxidationsmittel für das L-Cystein, ausgeführt wird.

2. Verfahren nach Anspruch 1, wobei als Enzym Tryptophansynthase verwendet wird.

## Revendications

1. Dans un procédé de production de L-cystine au départ de L-sérine, consistant à faire réagir la L-sérine en présence d'une enzyme de manière à former de la L-cystéine et à oxyder ladite L-cystéine, le perfectionnement selon lequel ladite L-sérine est mise à réagir avec une substance choisie dans le groupe formé par le sulfure d'hydrogène, des sulfures de métaux alcalins et des hydrogénosulfures de métaux alcalins et selon lequel la réaction de ladite L-sérine avec ladite substance est conduite en présence de sulfoxyde de diméthyle employé comme agent oxydant de ladite L-cystéine.

2. Procédé comme exposé à la revendication 1, où ladite enzyme est la tryptophane synthase.